Europäisches Patentamt

European Patent Office

Office européen des brevets

(19)

(11) **EP 1 118 962 A1**

(12) **EUROPÄISCHE PATENTANMELDUNG**

(43) Veröffentlichungstag:
**25.07.2001 Patentblatt 2001/30**

(51) Int Cl.$^7$: **G06T 7/00**

(21) Anmeldenummer: **01200029.5**

(22) Anmeldetag: **09.01.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **18.01.2000 DE 10001709**

(71) Anmelder:
• **Philips Corporate Intellectual Property GmbH 52064 Aachen (DE)**
Benannte Vertragsstaaten:
**DE**
• **Koninklijke Philips Electronics N.V. 5621 BA Eindhoven (NL)**
Benannte Vertragsstaaten:
**FR GB NL**

(72) Erfinder:
• **Grass, Michael, Dr. c/o Philips Corp.I.P.GmbH 52064 Aachen (DE)**
• **Kemkers, R., c/o Philips Corp. Intell. Pty GmbH 52064 Aachen (DE)**

(74) Vertreter: **Volmer, Georg, Dipl.-Ing.
Philips Corporate Intellectual Property GmbH, Habsburgerallee 11
52064 Aachen (DE)**

(54) **Registrierung von aufeinanderfolgenden tomographischen Datensätzen zur Bewegungsartefaktreduktion**

(57) Die Erfindung betrifft ein Verfahren zur Erstellung von Röntgenbildern (B) aus mindestens zwei Serien von Projektionsdatensätzen ($P_1$, $P_2$), welche nacheinander entlang unterschiedlicher Trajektorien ($T_1$, $T_2$) ermittelt wurden, wobei aus den Serien von Projektionsdatensätzen ($P_1$, $P_2$) jeweils ein 3D-Datensatz ($S_1$, $S_2$) erstellt wird. Um Bewegungen des Patienten zwischen der Erfassung der einzelnen Serien von Projektionsdatensätzen bei der Kombination der 3D-Datensätze zur Erstellung von möglichst artefaktfreien Röntgenbildern auszugleichen, wird erfindungsgemäß vorgeschlagen, eine die relative räumliche Lage der 3D-Datensätze ($S_1$, $S_2$) zueinander beschreibende Transformationsvorschrift (F) dadurch zu bestimmen, dass Voxel in einem 3D-Datensatz ($S_1$) ausgewählt und deren Lage in den anderen 3D-Datensätzen ($S_2$) mittels eines geeigneten Ähnlichkeitsmaßes bestimmt werden, und anschließend Röntgenbilder (B) aus den mittels der Transformationsvorschrift (F) kombinierten 3D-Datensätze ($S_1$, $S_2$) zu erstellen. Dadurch können sowohl abzubildende Phantomkörper für eine Feinjustierung der einzelnen 3D-Datensätze als auch manuelle Feinjustierungsschritte entfallen. Die Erfindung betrifft außerdem eine entsprechend ausgestaltete Röntgeneinrichtung.

FIG. 1

**Beschreibung**

Die Erfindung betrifft ein Verfahren zur Erstellung von Röntgenbildern aus mindestens zwei Serien von Projektionsdatensätzen, welche nacheinander entlang unterschiedlicher Trajektorien ermittelt wurden, wobei aus den Serien von Projektionsdatensätzen jeweils ein 3D-Datensatz erstellt wird. Die Erfindung betrifft weiter eine Röntgeneinrichtung, die insbesondere für die Durchführung dieses Verfahrens ausgestaltet ist.

Ein solches Verfahren und eine solche Anordnung sind aus der EP 860 696 A2 bekannt. Dort werden mittels einer C-Bogen-Röntgeneinrichtung zwei Serien von Projektionsdatensätzen entlang zweier halbkreisförmiger Trajektorien erfaßt, die unter einem Winkel von 60° zueinander liegen. Jede Serie von Projektionsdatensätzen bildet dann einen 3D-Datensatz, aus dem jeweils ein Rekonstruktionsbild erstellt werden kann. Da ein einzelner 3D-Datensatz nicht ausreichend Daten für eine vollständige und korrekte Rekonstruktion aufweist und sich bei der Rekonstruktion Artefakte ergeben, werden die beiden (oder mehrere) 3D-Datensätze durch gewichtete Addition kombiniert. Aus dem sich ergebenden Datensatz werden dann die gewünschten Bilder durch Rekonstruktion erstellt, in denen Artefakte in geringerem Maße auftreten.

Die Erfassung der Serien von Projektionsdatensätzen entlang der unterschiedlichen Trajektorien erfolgt normalerweise zeitlich nacheinander. Damit die Projektionsdatensätze bzw. die daraus erstellen 3D-Datensätze bei der anschließenden Kombination und Rekonstruktion optimal zusammenpassen, wäre es erforderlich, dass sich das Untersuchungsobjekt, z.B. der Patient, während der Datenerfassung nicht bewegt. Insbesondere sollte die Lage des Untersuchungsobjekts bei der Erfassung der einzelnen Serien von Projektionsdatensätzen immer identisch sein, und es sollte eine möglichst geringe Translations- oder Rotationsbewegung des Untersuchungsobjekts auftreten. Da sich dies in der Praxis bei der Untersuchung eines Patienten kaum zu 100 % realisieren läßt, ist es auch bekannt, beispielsweise einen Phantomkörper bei der Erfassung der Projektionsdatensätze in den Röntgenbildern mit abzubilden, der anschließend zur Feinjustierung benutzt werden kann, um die 3D-Datensätze in Übereinstimmung zu bringen (engl. "matching"). Dies erfolgt durch einen Benutzer.

Der Erfindung liegt deshalb die Aufgabe zugrunde, ein Verfahren anzugeben, mit dem sich 3D-Datensätze kombinieren lassen, ohne dass von einem Benutzer eine Feinjustierung vorgenommen werden muß. Außerdem soll eine geeignet ausgestaltete Röntgeneinrichtung angegeben werden.

Diese Aufgabe wird durch ein Verfahren gemäß Anspruch 1 bzw. durch eine Röntgeneinrichtung gemäß Anspruch 6 gelöst.

Die Erfindung geht dabei von der Erkenntnis aus, dass in allen 3D-Datensätzen dasselbe Untersuchungsobjekt abgebildet ist und sich deshalb einzelne Strukturen in allen 3D-Datensätzen wiederfinden lassen. Dies wird erfindungsgemäß dazu ausgenutzt, in einem ersten 3D-Datensatz die Voxel-Bildwerte wenigstens eins Subvolumens auszuwählen und in den anderen 3D-Datensätzen zu suchen, um daraus eine Transformationsvorschrift abzuleiten, die eine eventuelle Translations- oder Rotations-Bewegung zwischen der Erstellung einzelner 3D-Datensätze beschreibt. Die Auswahl des Subvolumens $V_2$ erfolgt dabei im allgemeinen automatisch. Bei der Suche von in einem ersten 3D-Datensatz ausgewählten Voxeln in den anderen 3D-Datensätzen wird ein geeignetes Ähnlichkeitsmaß verwendet, um iterativ das entsprechende Voxel in den anderen 3D-Datensätzen zu finden.

Je nach der gewünschten Genauigkeit kann dieses Verfahren mit entsprechend vielen Voxeln durchgeführt werden, die möglichst über das gesamte durch den 3D-Datensatz dargestellte Volumen verteilt sein sollten. Die gefundene Transformationsvorschrift bzw. mehrere Transformationsvorschriften werden dann dazu benutzt, um Bewegungen des Untersuchungsobjekts zu korrigieren und die 3D-Datensätze quasi in Übereinstimmung zu bringen, zu einem kompletten Datensatz zu kombinieren und daraus die gewünschten Bilder zu erstellen. Dies ist bei dem erfindungsgemäßen Verfahren ohne Verwendung eines mit in den Röntgenbildern abzubildenden Phantomobjektes oder anderer Marker möglich und kann automatisch, d.h. ohne Eingriffe eines Benutzers, erfolgen.

Vorteilhafterweise werden gemäß den Ansprüchen 2 und 3 zur Bestimmung der Transformationsvorschrift mehrere Voxel in jeweils einem Teilvolumen eines 3D-Datensatzes und/oder einzelne signifikante Bildinformationen enthaltende Voxel ausgewählt. Diese Auswahl kann jeweils automatisiert sein oder auch manuell von einem Benutzer interaktiv vorgenommen werden.

Bevorzugt werden die in Anspruch 4 angegebenen Funktionen als Ähnlichkeitsmaß verwendet. Es sind jedoch auch andere Möglichkeiten denkbar.

Das erfindungsgemäße Verfahren findet primär Anwendung bei einer C-Bogen-Röntgeneinrichtung, ist jedoch auch bei einer Computertomographieeinrichtung anwendbar, insbesondere kann die Erfindung auch bei einer Röntgeneinrichtung oder einem Computertomographen mit kegelförmigem Röntgenstrahlenbündel eingesetzt werden.

Eine erfindungsgemäße Röntgeneinrichtung mit einer Röntgenquelle, einem Röntgendetektor, einer Rekonstruktionseinheit und einer Recheneinheit ist in Anspruch 6 angegeben.

Die Erfindung wird nachfolgend anhand der Zeichnungen näher erläutert. Es zeigen:

Fig. 1 eine erfindungsgemäße C-Bogen-Röntgeneinrichtung,

Fig. 2 eine Skizze zur Erläuterung zweier Trajektorien,

Fig. 3 ein Blockdiagramm zur Erläuterung des erfindungsgemäßen Verfahrens und

Fig. 4 eine erfindungsgemäß ausgestaltete Computertomographieeinrichtung.

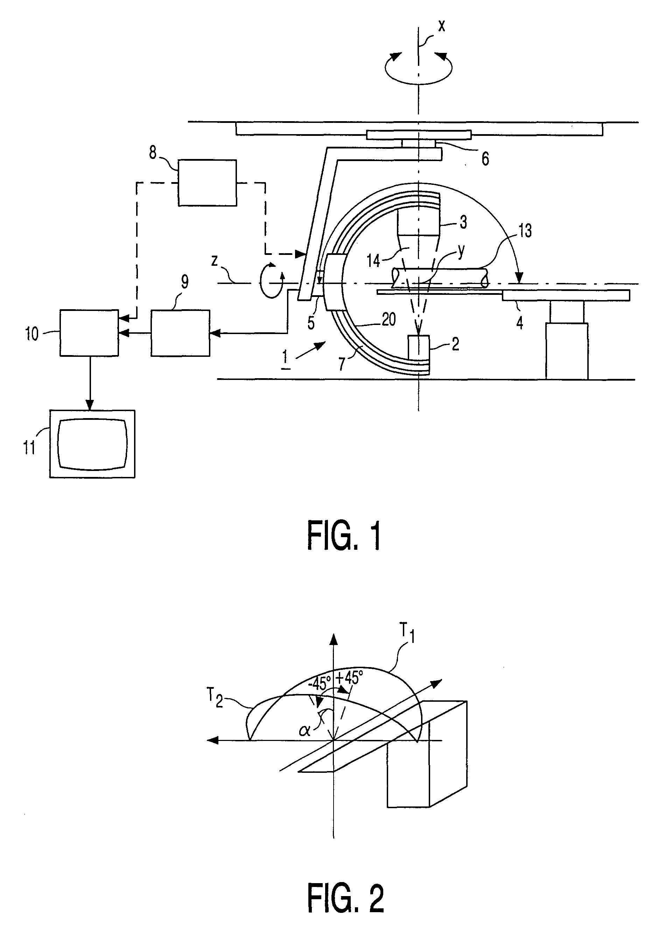

Die in Fig. 1 gezeigte C-Bogen-Röntgeneinrichtung 1 weist eine an einem Ende des C-Bogens 20 angeordnete Röntgenröhre 2 und einen am anderen Ende des C-Bogens 20 angeordneten Röntgendetektor 3 auf. Die Röntgenröhre 2 erzeugt ein kegelförmiges Röntgenstrahlenbündel 14, das ein im Untersuchungsbereich auf einem Patiententisch 4 angeordnetes Untersuchungsobjekt 13, z.B. einen Patienten, durchsetzt und danach auf den zweidimensionalen Röntgendetektor 3 trifft. Röntgenröhre 2 und Röntgendetektor 3 sind mittels der am C-Bogen 20 angebrachten Schienen 7 um die y-Achse rotierbar. Aufgrund der Aufhängung mittels mehrerer Arme und Gelenke 5, 6 ist die Position des C-Bogens 20 in verschiedenen Richtungen veränderbar, beispielsweise kann der C-Bogen 20 um die x-, y- und z-Achse rotieren.

Die Steuerung dieser Bewegungen zur Erfassung von Projektionen aus unterschiedlichen Röntgenpositionen und die Steuerung der Datenerfassung erfolgen mittels einer Steuereinheit 8. Die ermittelten Projektionen werden an eine Rekonstruktionseinheit 9 weitergegeben, die aus einer Serie von entlang einer Trajektorie erfaßten Projektionen jeweils einen 3D-Datensatz und daraus ggf. ein Rekonstruktionsbild erstellt. Diese 3D-Datensätze bzw. die Rekonstruktionsbilder werden anschließend einer Recheneinheit 10 zugeführt, die nach dem erfindungsgemäßen Verfahren die Transformationsvorschriften (bzw Transformationsparameter für eine Transformation) zwischen den einzelnen 3D-Datensätzen bestimmt und schließlich aus den 3D-Datensätzen mit Hilfe der Transformationsvorschriften die gewünschten Röntgenbilder erstellt, welche wiederum auf einem Monitor 11 dargestellt werden können.

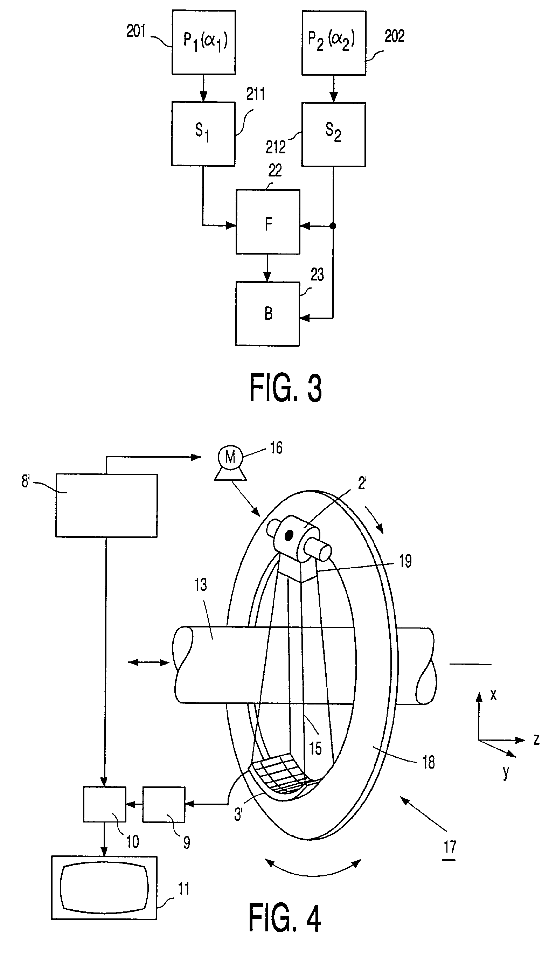

Eine Skizze zweier Trajektorien $T_1$ und $T_2$ ist in Fig. 2 gezeigt. Jede Trajektorie beschreibt dabei den Weg, den der Mittelpunkt der Detektorfläche des Röntgendetektors 3 während der Erfassung von Projektionsdatensätzen zurücklegt. Die Trajektorie ist also die Kurve durch alle Röntgenpositionen, in denen jeweils eine Projektion erfaßt wird. Im gezeigten Fall beschreiben die Trajektorien $T_1$ und $T_2$ jeweils einen Halbkreis und sind um einen Winkel $2\alpha=90°$ zueinander verkippt. Aus den entlang der Trajektorie $T_1$ erfaßten Projektionen wird ein erster 3D-Datensatz, aus den entlang der Trajektorie $T_2$ erfaßten Projektionen ein zweiter 3D-Datensatz erstellt. Um diese Datensätze in Übereinstimmung zu bringen, d.h. eine ggf. aufgetretene Translations- oder Rotationsbewegung des Patienten zwischen der Erfassung der ersten und der zweiten Serie von Projektionen auszugleichen, wird anschließend die Transformationsvorschrift zwischen den beiden 3D-Datensätzen bestimmt, was anhand von Fig. 3 näher erläutert werden soll.

Bei dem in Fig. 3 gezeigten Blockdiagramm sind zunächst in den Blöcken 201und 202 symbolisch zwei Sätze von Projektionen $P_1(\alpha_1)$ und $P_2(\alpha_2)$ als Ausgangspunkte dargestellt, die entlang zweier unter den Winkeln $\alpha_1$ bzw. $\alpha_2$ zu einer Bezugsebene verlaufenden Trajektorien $T_1$ bzw. $T_2$ ermittelt wurden. Aus jedem dieser Projektionsdatensätze $P_1$, $P_2$ wird ein 3D-Datensatz $S_1$, $S_2$ in den Blöcken 211 und 212 erstellt.

Aus diesen 3D-Datensätzen $S_1$, $S_2$ wird anschließend im Block 22 eine Transformationsvorschrift bestimmt und auf einen der beiden Datensätze (oder beide) angewandt - im Beispiel auf $S_1$.

Die Transformationsvorschrift wird z.B. wie folgt ermittelt:

a) Aus einem der beiden 3D-Datensätze, z.B. dem Datensatz $S_1$, werden die Voxel (z.B. 16 x 16 x 16) eines Subvolumens $V_1$ (d. h. eines Teils des Volumens, das durch den 3D-Datensatz abgebildet wird) ausgewählt. Diese Auswahl kann automatisch erfolgen, beispielsweise in dem ein Subvolumen mit möglichst hohem Kontrast ausgewählt wird (bei dem die Voxel-Bildwerte in den Subvolumen möglichst stark von ihrem Mittelwert abweichen).

b) Dann werden die Koordinaten $x_1$ der Voxel in dem Subvolumen $V_1$ einer Transformation unterzogen, z.B gemäß der Beziehung

$$x_2 = \mathfrak{R}(x_1 - u) + t \tag{1}$$

wobei $x_1$, $x_2$, u, t Vektoren sind und $\mathfrak{R}$ eine Rotationsmatrix ist, die die Transformation der Koordinaten bei einer Drehung des Koordinatensystems um seinen Ursprung beschreibt. Von den Vektoren ist lediglich der Vektor $x_1$ bekannt (es ist der Vektor, der das Voxel mit dem Koordinatenursprung verbindet). Der Vektor u stellt die Koordinaten des Punktes dar, um den die Drehung erfolgt, und t ist ein Vektor, der der Translation des Voxels enspricht. Der resultierende Vektor $x_2$ stellt die Koordinaten des Voxels in dem durch den zweiten 3D-Datensatz repräsentierten Volumen dar. Wendet man die Transformation auf sämtlich Voxel des Subvolumens an, dann ergibt sich ein gleich großes Subvolumen $V_2$ im zweiten Datensatz $S_2$.

c) Anschließend wird die Übereinstimmung der Voxel-Bildwerte des Subvolumens $V_2$ mit den Voxel-Bildwerten des Subvolumens $V_1$ aus dem ersten 3D-Datensatz $S_1$ durch ein Ähnlichkeitsmaß bewertet. Danach wird die Position und/oder die Orientierung des im zweiten Datensatz ausgewählten Subvolumens variiert (indem u, t, oder $\Re$ variiert wird), und die Ähnlichkeit dieses Subvolumens mit dem Subvolumen $V_1$ wird erneut durch das Ähnlichkeitsmaß bewertet. Diese Schritte werden iterativ so lange wiederholt, bis aus dem 3D-Datensatz $S_2$ das Subvolumen gefunden wurde, das am besten mit dem Subvolumen $V_1$ des 3D-Datensatzes $S_1$ übereinstimmt. Die zugehörigen Transformationsparameter (u, t, oder $\Re$) definieren dann die Transformationsvorschrift.

Als Ähnlichkeitsmaß kann man z.B. die mittlere absolute Differenz MAD der Voxel-Bildwerte in den beiden Volumina betrachten:

$$\mathrm{MAD} = \frac{1}{n} \sum_{i=1}^{n} (V_{1i} - V_{2i})$$

wobei n die Zahl der Voxel in dem Subvolumen $V_1$ bzw. $V_2$ ist und $V_{1i}$ bzw. $V_{2i}$ den i-ten Voxel-Bildwert im ersten Subvolumen $V_1$ bzw. im zweiten Subvolumen $V_2$ darstellen. Anstatt die mittlere absolute Differenz zu minimieren, kann man auch beispielsweise die Wurzel der quadratischen Differenzen minimieren oder die Ähnlichkeit mit einem geeigneten Korrelationskoeffizienten (z.B. für eine Kreuzkorrelation, Doppelte Korrelation oder die Pearsonsche lineare Korrelation) bewerten.

Die Ableitung der Transformationsparameter aus einem Subvolumen benötigt weniger Rechenzeit, als wenn man diese Parameter unter Zuhilfenahme sämtlicher Voxel-Bildwerte der 3D-Datensätze bestimmen würde. Allerdings ist sie weniger genau und durch Rauschen stärker beeinflußt werden. Die Genauigkeit kann verbessert werden, indem man pro 3D-Datensatz zwei oder mehr Subvolumina heranzieht und über die für die verschiedenen Subvolumina gefundenen Transformationsparameter mittelt.

Die vorstehend erläuterte Transformation beruht auf der Annahme, dass sich in dem Untersuchungsbereich ein starres Untersuchungsobjekt befindet. Das Objekt könnte aber auch verformbar sein. Die ortsabhängigen Transformationsparameter könnten dann mit Hilfe eines so genannten "elastic matching" - Verfahrens bestimmt werden.

Aus dem transformierten 3D-Datensatz $S_1$ und aus dem Datensatz $S_2$ wird im Block 23 mit Hilfe einer vorzugsweise gewichteten Summierung der Voxel-Bildwerte von zueinander entsprechend der Transformation korrespondierenden Voxeln ein verbesserter 3D-Datensatz S bestimmt. Wenn der Gewichtungsfaktor, mit dem dabei ein Voxel-Bildwert multipliziert wird, umso größer ist, je geringer sein Abstand von der durch die zugehörige Trajektorie $T_1$ bzw. $T_2$ definierten Ebene ist (und umgekehrt), desto geringer werden das Rauschen und die Artefakte. Die Artefakte in den beiden 3D-Datensätzen $S_1$ und $S_2$ machen sich in den Voxeln, die relativ weit von der genannten Ebene entfernt sind, nämlich stärker bemerkbar.

Eine erfindungsgemäße Computertomographieeinrichtung 17 ist in Fig. 4 gezeigt. Die Röntgenquelle 2' mit einem Kollimator 19 zur Erzeugung eines kegelförmigen Röntgenstrahlenbündels 15 und der Röntgendetektor 3' sind dabei an einer ringförmigen Gantry 18 angeordnet und rotieren zur Erfassung von Projektionen um das entlang der z-Achse angeordnete Untersuchungsobjekt 13. Dazu wird die Gantry 18 von einer Motorsteuerung 16 gesteuert, die wiederum von einer Steuereinheit 8' gesteuert wird. Die erfaßten Projektionen werden an eine Rekonstruktionseinheit 9 zur Erstellung von 3D-Datensätzen und Rekonstruktionsbildern weitergegeben, die wiederum der Recheneinheit 10 zugeleitet werden. Die Ermittlung der Transformationsvorschrift und die anschließende Erstellung von Röntgenbildern erfolgt im Zusammenhang mit der C-Bogen-Röntgeneinrichtung 1 wie oben erläutert, weshalb auf eine weitere Beschreibung hier verzichtet werden soll.

Die gezeigten Röntgeneinrichtungen sind lediglich beispielhafte Ausführungsformen der Erfindung. Die Erfindung kann auch bei anderen Röntgeneinrichtungen eingesetzt werden, bei denen aus mehreren 3D-Datensätzen durch Kombination ein kompletter Datensatz und daraus Röntgenbilder erstellt werden sollen. Auch die in Fig. 2 gezeigten Trajektorien sowie deren Anzahl sind lediglich beispielhaft. Die Erfassung von Projektionen kann auch entlang anderer und mehr als zwei Trajektorien beispielsweise zweier oder mehrerer paralleler Vollkreise oder zweier senkrecht aufeinander stehender Vollkreise erfolgen.

**Patentansprüche**

1. Verfahren zur Erstellung von Röntgenbildern (B) aus mindestens zwei Serien von Projektionsdatensätzen ($P_1$, $P_2$), welche nacheinander entlang unterschiedlicher Trajektorien ($T_1$, $T_2$) ermittelt wurden, wobei aus den Serien von Projektionsdatensätzen ($P_1$, $P_2$) jeweils ein 3D-Datensatz ($S_1$, $S_2$) erstellt wird, wobei eine die relative räumliche Lage der 3D-Datensätze ($S_1$, $S_2$) zueinander beschreibende Transformationsvorschrift (F) dadurch bestimmt wird, dass Voxel in einem 3D-Datensatz ($S_1$) ausgewählt und deren Lage in den anderen 3D-Datensätzen ($S_2$)

mittels eines geeigneten Ähnlichkeitsmaßes bestimmt werden, und wobei Röntgenbilder (B) aus den mittels der Transformationsvorschrift (F) kombinierten 3D-Datensätzen ($S_1$, $S_2$) erstellt werden.

2. Verfahren nach Anspruch 1,
dadurch gekennzeichnet,
dass zur Bestimmung der Transformationsvorschrift (F) mehrere Voxel in jeweils einem Teilvolumen eines 3D-Datensatzes ($S_1$, $S_2$) ausgewählt werden.

3. Verfahren nach Anspruch 1,
dadurch gekennzeichnet,
dass zur Bestimmung der Transformationsvorschrift (F) einzelne, signifikante Bildinformationen enthaltende Subvolumina ($V_1$, $V_2$) ausgewählt werden.

4. Verfahren nach Anspruch 1,
dadurch gekennzeichnet,
dass als Ähnlichkeitsmaß die mittlere absolute Differenz, die mittlere quadratische Differenz, die doppelte Korrelation oder die Pearsonsche lineare Korrelation verwendet wird.

5. Verfahren nach Anspruch 1,
dadurch gekennzeichnet,
dass die Projektionsdatensätze ($P_1$, $P_2$) mittels einer C-Bogen- Röntgeneinrichtung (1) oder einer Computertomographieeinrichtung (17) ermittelt werden.

6. Röntgeneinrichtung, insbesondere zur Durchführung des Verfahrens nach Anspruch 1, mit einer Röntgenquelle (2) und einem Röntgendetektor (3) zur Erfassung von mehreren Serien von Projektionsdatensätzen ($P_1$, $P_2$) entlang unterschiedlicher Trajektorien ($T_1$, $T_2$) um ein Untersuchungsobjekt (13), mit einer Rekonstruktionseinheit (9) zur Erstellung von 3D-Datensätzen($S_1$, $S_2$) aus jeweils einer Serie von Projektionsdatensätzen ($P_1$, $P_2$) und mit einer Recheneinheit (10), welche derart ausgestaltet ist, dass eine die relative räumliche Lage der 3D-Datensätze ($S_1$, $S_2$) zueinander beschreibende Transformationsvorschrift (F) dadurch bestimmt wird, dass Voxel in einem 3D-Datensatz ($S_1$) ausgewählt und deren Lage in den anderen 3D-Datensätzen ($S_2$) mittels eines geeigneten Ähnlichkeitsmaßes bestimmt werden, und dass Röntgenbilder (B) aus den mittels der Transformationsvorschrift (F) kombinierten 3D-Datensätze ($S_1$, $S_2$) erstellt werden

7. Röntgeneinrichtung nach Anspruch 6,
dadurch gekennzeichnet,
dass die Röntgeneinrichtung eine C-Bogen-Röntgeneinrichtung (1) oder eine Computertomographieeinrichtung (17) ist.

FIG. 1

FIG. 2

FIG. 3

FIG. 4

**Europäisches Patentamt**

**EUROPÄISCHER RECHERCHENBERICHT**

Nummer der Anmeldung

EP 01 20 0029

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | US 5 647 360 A (BANI-HASHEMI ALI REZA ET AL) 15. Juli 1997 (1997-07-15)<br>* Abschnitt "Summary of the Invention" in Spalten 3 und 4 *<br>* Spalte 8, Zeile 54 - Zeile 56 *<br>--- | 1-7 | G06T7/00 |
| Y | GRASS M ET AL: "Three-dimensional reconstruction of high contrast objects using C-arm image intensifier projection data"<br>COMPUTERIZED MEDICAL IMAGING AND GRAPHICS, NOV.-DEC. 1999, ELSEVIER, UK,<br>Bd. 23, Nr. 6, Seiten 311-321,<br>XP001001414<br>ISSN: 0895-6111<br>* Zusammenfassung; Abbildung 2 *<br>* Seite 315, linke Spalte, Zeile 30 - Zeile 37 *<br>--- | 1-7 | |
| A | BROWN L G: "A SURVEY OF IMAGE REGISTRATION TECHNIQUES"<br>ACM COMPUTING SURVEYS,NEW YORK, NY,US,<br>Bd. 24, Nr. 4,<br>1. Dezember 1992 (1992-12-01), Seiten 325-376, XP000561460<br>ISSN: 0360-0300<br>sections 4.2 ad 4.3<br>--- | 4 | RECHERCHIERTE SACHGEBIETE (Int.Cl.7)<br><br>G06T<br>G01R |
| A | MAURER C R ET AL: "A REVIEW OF MEDICAL IMAGE REGISTRATION"<br>INTERACTIVE IMAGE GUIDED NEUROSURGERY,XX,XX,<br>1. September 1993 (1993-09-01), Seiten 17-44, XP000870293<br>* Seite 33, Absatz 2 *<br>----- | 4 | |

Der vorliegende Recherchenbericht wurde für alle Patentansprüche erstellt

| Recherchenort | Abschlußdatum der Recherche | Prüfer |
|---|---|---|
| BERLIN | 18. Mai 2001 | Bouchaâla, N |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

\& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 01 20 0029

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-05-2001

| Im Recherchenbericht angeführtes Patentdokument | Datum der Veröffentlichung | Mitglied(er) der Patentfamilie | Datum der Veröffentlichung |
|---|---|---|---|
| US 5647360 A | 15-07-1997 | KEINE | |

EPO FORM P0461

Für nähere Einzelheiten zu diesem Anhang : siehe Amtsblatt des Europäischen Patentamts, Nr.12/82